(19) Europäisches Patentamt
European Patent Office
Office européen des brevets

(11) Veröffentlichungsnummer: **0 044 410**
**B1**

(12) **EUROPÄISCHE PATENTSCHRIFT**

(45) Veröffentlichungstag der Patentschrift: **21.12.83**

(51) Int. Cl.³: **C 07 C 127/22,** A 01 N 47/34

(21) Anmeldenummer: **81104688.7**

(22) Anmeldetag: **19.06.81**

(54) **Substituierte N-Benzoyl-N'-phenoxyphenylharnstoffe, Verfahren zu ihrer Herstellung und ihre Verwendung zur Bekämpfung von Insekten.**

(30) Priorität: **16.07.80 DE 3026825**

(43) Veröffentlichungstag der Anmeldung: **27.01.82 Patentblatt 82/4**

(45) Bekanntmachung des Hinweises auf die Patenterteilung: **21.12.83 Patentblatt 83/51**

(84) Benannte Vertragsstaaten: **BE CH DE FR GB IT LI NL SE**

(56) Entgegenhaltungen:
**JOURNAL OF AGRICULTURAL AND FOOD CHEMISTRY, Band 21, Nr. 3, Mai/Juni 1973, Seiten 348-354 American Chemical Society Washington D.C., U.S.A. K. WELLINGA et al.: "Synthesis and laboratory evaluation of 1-(2,6-disubstituted benzoyl)-3-phenyl-ureas, a new class of insecticides. I. 1-(2,6-dichlorobenzoyl)-3-phenylureas"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, *Carl-Bosch-Strasse 38, D-6700 Ludwigshafen* (DE)**

(72) Erfinder: **Lange, Arno, Dr., Friedrichsplatz 11, D-6800 Mannheim (DE)**
Erfinder: **Kiehs, Karl, Dr., Sudetenstrasse 22, D-6840 Lampertheim (DE)**
Erfinder: **Adolphi, Heinrich, Dr., Kalmitweg 11, D-6703 Limburgerhof (DE)**

**EP 0 044 410 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Substituierte N-Benzoyl-N'-phenoxyphenylharnstoffe, Verfahren zu ihrer Herstellung und ihre Verwendung zur Bekämpfung von Insekten

Die Erfindung betrifft substituierte N-Benzoyl-N'-phenoxyphenylharnstoffe, ein Verfahren zu ihrer Herstellung und Insektizide, die diese Verbindungen als Wirkstoffe enthalten.

Es ist bereits bekannt, N-Benzoyl-N'-phenoxyphenylharnstoffe, z. B. N-(2,6-Dichlorbenzoyl)-N'-4-(4-chlor-phenoxy)-phenyl-harnstoff, als Insektizide zu verwenden (J. Agr. Food Chem. 21, 348 [1973], DE-A-2 531 202).

Es wurde nun gefunden, daß substituierte N-Benzoyl-N'-phenoxyphenylharnstoffe der Formel

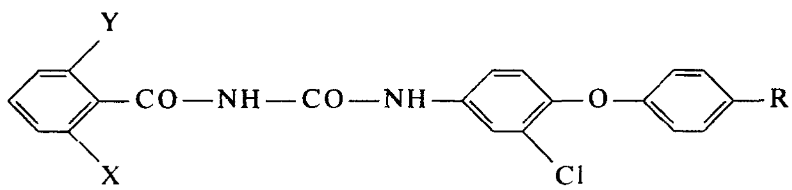

(I)

in der X und Y Fluor oder Chlor und R Fluor, Chlor oder Brom bedeuten, eine bessere insektizide Wirkung als bekannte N-Benzoyl-N'-phenoxyphenylharnstoffe besitzen.

Bevorzugt sind Verbindungen der Formel I, in denen X Fluor bedeutet.

Die neuen Verbindungen lassen sich herstellen, indem man

a) eine Verbindung der Formel

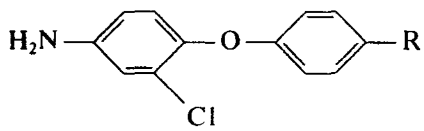

(II)

in der R die obengenannten Bedeutungen hat, mit einem Benzoylisocyanat der Formel

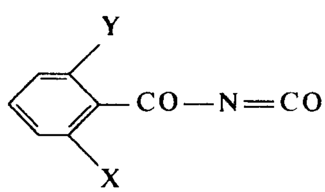

(III)

in der X und Y die obengenannten Bedeutungen haben, oder

b) eine Verbindung der Formel

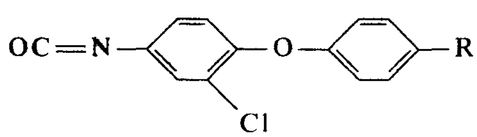

(IV)

in der R die obengenannten Bedeutungen hat, mit einem Benzamid der Formel

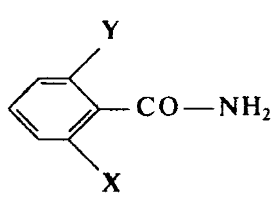

(V)

in der X und Y die obengenannten Bedeutungen haben,

umsetzt.

Die Verfahren a) und b) werden bevorzugt in Lösungs- bzw. Verdünnungsmitteln durchgeführt, wie in gegebenenfalls chlorierten Kohlenwasserstoffen, z. B. Benzol, Toluol, Xylol, Benzin, Methylenchlorid, Chloroform, Tetrachlorkohlenstoff, Chlorbenzol, in Ethern, z. B. Diethylether, Dibutylether, Dioxan, in Ketonen, z. B. Aceton, Methylethylketon, Methylisopropylketon, Methylisobutylketon, oder in Nitrilen, z. B. Acetonitril oder Benzonitril.

Die Reaktion erfolgt in der Regel bei Normaldruck und Temperaturen zwischen 0 und 120°C, vorzugsweise zwischen 20 und 50°C. Die Reaktionspartner werden vorzugsweise in äquimolaren Mengen eingesetzt. Es ist nicht erforderlich, die Ausgangsprodukte rein einzusetzen.

Die folgenden Beispiele erläutern die Erfindung:

## Beispiel 1

6,4 g 3-Chlor-4-(4-chlorphenoxy)-anilin werden bei Raumtemperatur in 50 ml Toluol gelöst. Dann werden 4,7 g 2,6-Difluorbenzoylisocyanat zugetropft, wobei die Temperatur um 15°C steigt. Danach rührt man eine Stunde lang bei 50°C nach, läßt abkühlen und saugt den Niederschlag ab. Man erhält 8,5 g N-(2,6-Difluorbenzoyl)-N'-[4-(4-chlorphenoxy)-3-chlorphenyl]-harnstoff vom Fp = 194 – 196°C.

Analog Beispiel 1 werden hergestellt:

2. N-(2,6-Difluorbenzoyl)-N'-[4-(4-fluorphenoxy)-3-chlorphenyl]-harnstoff; Fp = 159 – 161°C
3. N-(2,6-Difluorbenzoyl)-N'-[4-(4-bromphenoxy)-3-chlorphenyl]-harnstoff; Fp = 182 – 186°C
4. N-2,6-Dichlorbenzoyl-N'-[4-(4-bromphenoxy)-3-chlorphenyl]-harnstoff; Fp = 208 – 210°C
5. N-(2,6-Dichlorbenzoyl)-N'-[4-(4-chlorphenoxy)-3-chlorphenyl]-harnstoff; Fp = 186 – 189°C
6. N-(2,6-Dichlorbenzoyl)-N'-[4-(4-fluorphenoxy)-3-chlorphenyl]-harnstoff; Fp = 183 – 185°C
7. N-(2-Chlor-6-fluor-benzoyl)-N'-[4-(4-chlorphenoxy)-3-chlor-phenyl]-harnstoff; Fp = 173 – 175°C
8. N-(2-Chlor-6-fluor-benzoyl)-N'-[4-(4-fluorphenoxy)-3-chlor-phenyl]-harnstoff; Fp = 179 – 183°C
9. N-(2-Chlor-6-fluor-benzoyl)-N'-[4-(4-bromphenoxy)-3-chlor-phenyl]-harnstoff; Fp = 179 – 181°C

Die erfindungsgemäßen Verbindungen der Formel I sind geeignet, Insekten wirksam zu bekämpfen. Sie sind als Adultizide und Ovizide geeignet.

Zu den schädlichen Insekten gehören aus der Ordnung der Schmetterlinge (Lepidoptera) beispielsweise

Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattrum), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise

Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spagelhähnchen), Lema melanopus (Getreidehähnchen), Leptinotarsa decemlineata (Kartoffelkäfer), Phaedon cochleariae (Meerrettich-Blattkäfer), Phyllotetra nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus

pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Stiophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise

Lycoria pectorialis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise

Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);

aus der Ordnung der Wanzen (Heteroptera) beispielsweise

Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise

Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Cysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Terminten (Isoptera) beispielsweise

Reticulitermes lucifugus, Calotermes flavicollis, Leucotermes flavipes, Termes natalensis;

aus der Ordnung der Geradflügler (Orthoptera) beispielsweise

Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marikkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Die erfindungsgemäßen Verbindungen können mit Erfolg im Pflanzenschutz sowie auf dem Hygiene-, Vorratsschutz- und Veterinärsektor als Schädlingsbekämpfungsmittel eingesetzt werden. Dabei können sie als Kontakt- und Fraßgifte verwendet werden.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und

aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutynaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gew.-Teile des Wirkstoffes Nr. 1 werden mit 97 Gewichtsteilen feinteiligem Kaolin innig vermischt. Man erhält auf diese Weise in Stäubemittel, das 3 Gew.-% des Wirkstoffes enthält.

II. 30 Gewichtsteile des Wirkstoffes Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffes mit guter Haftfähigkeit.

III. 20 Gewichtsteile des Wirkstoffes Nr. 5 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffes enthält.

IV. 20 Gewichtsteile des Wirkstoffes Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffes enthält.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10 kg/ha, vorzugsweise 0,5 bis 2,0 kg/ha.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1

zugemischt werden.

Beispielsweise können folgende Mittel zugemischt werden:

1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methyl-mercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxy]-thio-acetimidat, Methyl-N',N'-dimethyl-N-[(methylcarbamoyl)oxy]-1-thiooxamidat, N-(2-Methyl-4-chlor-phenyl)-N',N'-dimethylformamidin, Tetrachlorthiophen, ı-(2,6-Difluor-benzoyl-3-(4-chlor-phenyl)-harnstoff O,O-Dimethyl-O-(p-ıitrophenyl)-phosphorthioat, O,O-Diethyl-O-(p-nitrophenyl)-phosphortioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-iodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl)-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methyl-carbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthio-methyl)-phosphordithioat, O,O-Diethyl-S-[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[4,6-diamino-1,3,5-triazin-2-yl)-methyl]-phosphordithioat,

O,O-Diethyl-(1-phenyl-1,2,4-triazol-3-yl)-thionophosphat,
O,S-Dimethyl-phosphor-amido-thioat, O,S-Dimethyl-N-acetyl-phosphoramidothioat,
$\gamma$-Hexachlorcyclohexan, 1,1-Di-(p-methoxyphenyl)-2,2,2-trichlor-ethan,
6,7,8,9,10,10-Hexachloro-1,5,5a,6,9,9a-hexahydro-6,9-methano-2,4,3-benzodioxa-thiepin-3-oxid,
Pyrethrine, DL-2-Allyl-3-methyl-cyclopenten-(2)-on-(1)-yl-(4)-DL-cis,-trans-chrysanthemat,
5-Benzyl-furyl-(3)-methyl-DL-cis,-trans-chrysanthemat,
3-Phenoxybenzyl($\pm$)-cis,trans-2,2-dimethyl-3-(2,2-dichlorvinyl)-cyclopropancarboxylat,
$\alpha$-Cyano-3-phenoxybenzyl($\pm$)-cis,trans-2,2-dimethyl-3-(2,2-dichlorvinyl)-cyclopropancarboxylat,
(s)-$\alpha$-Cyano-3-phenoxybenzyl-cis(1R,3R)-2,2-dimethyl-3-(2,2-dibromvinyl)-cyclopropancarboxylat,
3,4,5,6-Tetrahydrophthalimidoethyl-DL-cis,trans-chrysanthemat,
2-Methyl-5-(2-propinyl)-3-furylmethyl-chrysanthemat,
($\alpha$-Cyano-3-phenoxybenzyl)-$\alpha$-isopropyl-4-chlorphenylacetat.

Die folgenden Beispiele belegen die biologische Wirkung der neuen Verbindungen. Vergleichsmittel sind die aus der DE-A-2 531 202 bekannten Wirkstoffe N-(2-Methylbenzoyl)-N'-[4-(4-chlorphenoxy)-3-chlorphenyl]-harnstoff (I) und N-(2-Fluorbenzoyl)-N'-[4-(4-chlorphenoxy)-3-chlorphenyl]-harnstoff (II).

Beispiel A

Zuchtversuch mit Moskito-Larven (Aedes aegypti)

200 ml Leitungswasser werden mit der Wirkstoffaufbereitung versetzt und darauf mit 30 bis 40 Aedes-Larven im 4. Stadium belegt. Die Versuchstemperatur beträgt 25° C. Beurteilt wird Verpuppung und Schlüpfen der Imagines, wobei eine unbehandelte Kontrolle als Maßstab dient. Während der Versuchsdauer wird einmal mit einem herkömmlichen pulverisierten Fischfutter gefüttert.

In diesem Test zeigen die Wirkstoffe Nr. 1 bis 9 eine bessere Wirkung als die Vergleichsmittel I und II.

Beispiel B

Zuchtversuch mit Stubenfliegen (Musca domestica)

50 g eines Nährbodens aus 100 Teilen Wasser, 10 Teilen Bäckerhefe, 10 Teilen Trockenmilch und einem Teil Agar werden in warmem Zustand mit der wäßrigen Aufbereitung des Wirkstoffes gründlich durchmischt. Nach dem Erkalten belegt man den Nährboden mit ca. 0,1 ml Fliegeneiern und beobachtet deren Entwicklung über eine Woche. Die Versuchstemperatur liegt bei 20° C.

Beispiel C

Zuchtversuch mit Baumwollwanzen (Dysdercus intermedius)

50 g Baumwollsaat werden für 24 Stunden in der wäßrigen Wirkstoffaufbereitung eingequollen. Die überstehende Flüssigkeit wird abgeschüttet. Darauf bringt man 20 Wanzen im vorletzten Larvenstudium in 1-l-Gläser, deren Boden mit feuchtem Sand bedeckt ist. Diese Tiere erhalten 7 Tage lang die vorbehandelten Samen als ausschließliche Nahrung. Anschließend wird unbehandeltes Futter geboten.

Beispiel D

Zuchtversuch mit Reismehlkäfern (Tribolium castaneum)

10 g Weizenmehl, die mit der entsprechenden Wirkstoffmenge innig vermischt sind, werden in 250-ml-Glasflaschen gefüllt und mit 20 Reismehlkäfern belegt. Nach der Eiablage (14 Tage) siebt man die Käfer ab. Das Mehl wird in die Flaschen zurückgefüllt und lagert bis zum Schlüpfen der nächsten Käfergeneration bei +24° C.

In den Beispielen B bis D zeigen die neuen Verbindungen, insbesondere die Wirkstoffe Nr. 1 und 5, eine wesentlich bessere Wirkung als das Vergleichsmittel I.

**Patentansprüche**

1. Substituierte N-Benzoyl-N'-phenoxyphenylharnstoffe der Formel

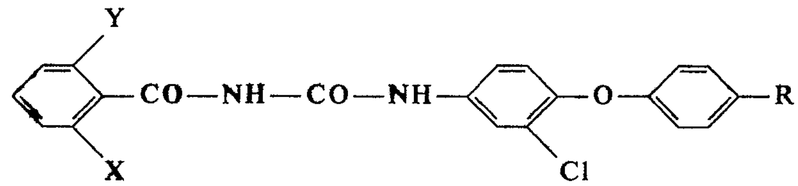

(I)

in der X und Y Fluor oder Chlor und R Fluor, Chlor oder Brom bedeuten.

2. N-(2,6-Difluorbenzoyl)-N'-[4-(4-chlorphenoxy)-3-chlor-phenyl]-harnstoff.

3. N-(2,6-Dichlorbenzyl)-N'-[4-(4-chlorphenoxy)-3-chlor-phenyl]-harnstoff.

4. Verfahren zur Herstellung von N-Benzoyl-N'-phenoxyphenyl-harnstoffen der Formel I gemäß Anspruch 1, dadurch gekennzeichnet, daß man

a) eine Verbindung der Formel

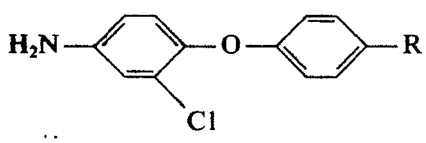

(II)

in der R die im Anspruch 1 genannten Bedeutungen hat, mit einem Benzoylisocyanat der Formel

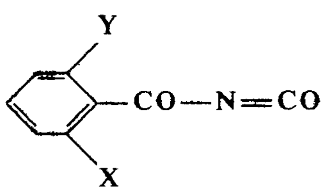

(III)

in der X und Y die im Anspruch 1 genannten Bedeutungen haben, oder

b) eine Verbindung der Formel

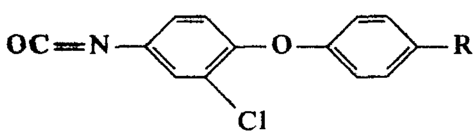

(IV)

in der R die im Anspruch 1 genannten Bedeutungen hat, mit einem Benzamid der Formel

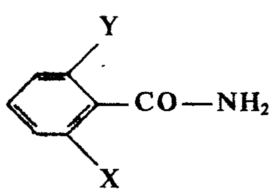

(V)

in der X und Y die im Anspruch 1 genannten Bedeutungen haben, umsetzt.

5. Insektizides Mittel, enthaltend einen N-Benzoyl-N'-phenoxyphenylharnstoff der Formel I gemäß Anspruch 1.

6. Insektizides Mittel, enthaltend inerte Zusatzstoffe und einen N-Benzoyl-N'-phenoxyphenylharnstoff der Formel I gemäß Anspruch 1.

7. Verfahren zur Bekämpfung von Insekten, dadurch gekennzeichnet, daß man eine insektizid wirksame Menge eines N-Benzoyl-N'-phenoxyphenylharnstoffes der Formel I gemäß Anspruch 1 auf die Insekten, ihre Eier und/oder ihren Lebensraum einwirken läßt.

8. Verfahren zur Herstellung eines insektiziden Mittels gemäß Anspruch 6, dadurch gekennzeichnet, daß man einen N-Benzoyl-N'-phenoxyphenylharnstoff der Formel I gemäß Anspruch 1 mit oberflächenaktiven Verbindungen und/oder Streckmitteln und/oder Synergisten mischt.

**Claims**

1. A substituted N-benzoyl-N′-phenoxyphenylurea of the formula

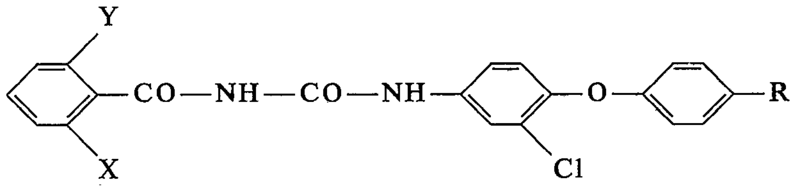

(I)

where X and Y denote fluorine or chlorine and R denotes fluorine, chlorine or bromine.

2. N-(2,6-difluorobenzoyl)-N′-[4-(4-chlorophenoxy)-3-chlorophenyl]-urea.

3. N-(2,6-dichlorobenzyl)-N′-[4-(4-chlorophenoxy)-3-chlorophenyl]-urea.

4. A process for the preparation of an N-benzoyl-N′-phenoxyphenylurea of the formula I as claimed in claim 1, wherein

a) a compound of the formula

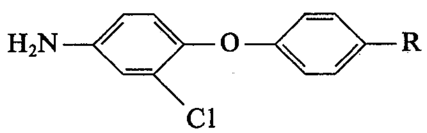

(II)

where R has the meanings given in claim 1, is reacted with a benzoyl isocyanate of the formula

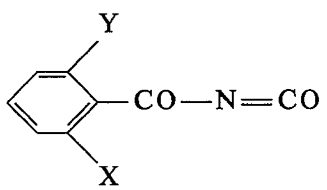

(III)

where X and Y have the above meanings, or

b) a compound of the formula

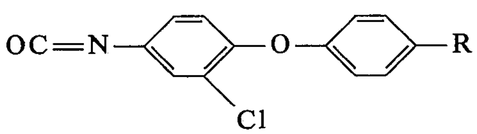

(IV)

where R has the meanings given in claim 1, is reacted with a benzamide of the formula

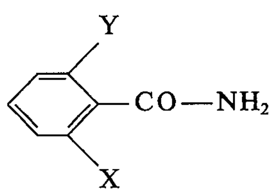

(V)

where X and Y have the meanings given in claim 1.

5. An insecticidal agent containing an N-benzoyl-N′-phenoxyphenylurea of the formula I as claimed in claim 1.

6. An insecticidal agent containing inert additives and an N-benzoyl-N′-phenoxyphenylurea of the formula I as claimed in claim 1.

7. A process for combating insects, wherein an insecticidally effective amount of an N-benzoyl-N′-phenoxyphenylurea of the formula I as claimed in claim 1 is allowed to act on the insects, their eggs and/or their habitat.

8. A process for producing an insecticidal agent as claimed in claim 6, wherein an N-benzoyl-N′-phenoxyphenylurea of the formula I as claimed in claim 1 is mixed with surface-active compounds and/or extenders and/or synergists.

**Revendications**

1. N-benzoyl-N'-phénoxyphénylurées substituées de formule

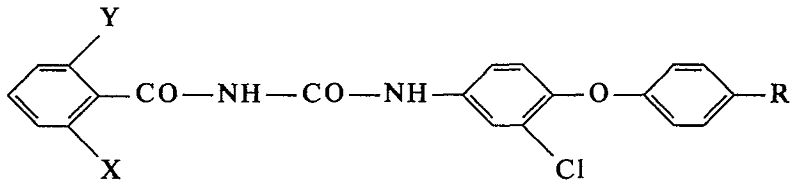

(I)

dans laquelle X et Y représentent fluor ou chlore et R fluor, chlore ou brome.

2. N-(2,6-dichlorbenzoyl)-N'-[4-(4-chlorophénoxy)-3-chlorophényl]-urée.

3. N-(2,6-dichlorobenzyl)-N'-[4-(4-chlorophénoxy)-3-chlorophényl]-urée.

4. Procédé de préparation de N-benzoyl-N'-phénoxyphénylurées, caractérisé par le fait que l'on fait réagir

a) un composé de formule

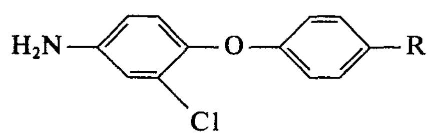

(II)

dans laquelle R a les significations données dans la revendication 1, avec un isocyanate de benzoyle de formule

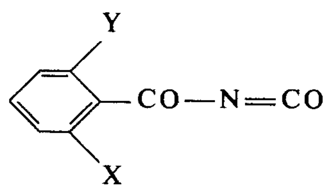

(III)

dans laquelle X et Y ont les significations indiquées dans la revendication 1, ou

b) un composé de formule

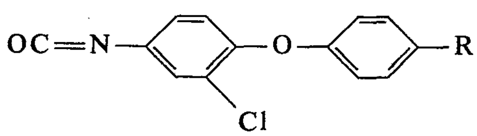

(IV)

dans laquelle R a les significations données dans la revendication 1, avec un benzamide de formule

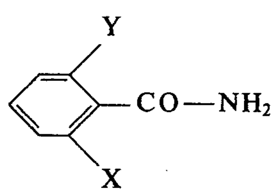

(V)

dans laquelle X et Y ont les significations indiquées dans la revendication 1.

5. Agent insecticide, contenant un N-benzoyl-N'-phénoxyphénylurée de formule I selon la revendication 1.

6. Agent insecticide contenant un additif inerte et un N-benzoyl-N'-phénoxyphénylurée de formule I selon la revendication 1.

7. Procédé de lutte contre les insectes caractérisé par le fait qu'on fait réagir sur les insectes, leurs œufs et/ou leur biotope, une quantité efficace, au point de vue insecticide, d'un N-benzoyl-N'-phénoxyphénylurée de formule I selon la revendication 1.

8. Procédé de préparation d'un agent insecticide selon la revendication 6, caractérisé par le fait qu'on mélange un N-benzoyl-N'-phénoxyphénylurée de formule I selon la revendication 1 avec des composés surfactifs et/ou diluants et/ou synergistes.